(19) Europäisches Patentamt
European Patent Office
Office européen des brevets

(11) Numéro de publication: **0 338 893 B1**

(12) **FASCICULE DE BREVET EUROPEEN**

(45) Date de publication de fascicule du brevet: **26.01.94**

(51) Int. Cl.5: **C07C 323/09**, C07C 323/31

(21) Numéro de dépôt: **89401010.7**

(22) Date de dépôt: **12.04.89**

# (54) Procédé de préparation de (+)-(2S,3S) (Amino-2 phényl) thio-3 hydroxy-2 (methoxy-4 phényl)-3 propionates de méthyle.

(30) Priorité: **19.04.88 FR 8805132**

(43) Date de publication de la demande:
**25.10.89 Bulletin 89/43**

(45) Mention de la délivrance du brevet:
**26.01.94 Bulletin 94/04**

(84) Etats contractants désignés:
**AT BE CH DE ES FR GB GR IT LI LU NL SE**

(56) Documents cités:
**EP-A- 0 158 339**
**EP-A- 0 182 273**
**US-A- 4 416 819**

**PATENT ABSTRACTS OF JAPAN, vol. 10, no. 339 (C-385)(2395), 15 novembre 1986; & JP - A - 61145160**

**PATENT ABSTRACTS OF JAPAN, vol. 10, no. 339 (C-385)(2395), 15 novembre 1986; & JP - A - 61145159**

(73) Titulaire: **SYNTHELABO**
**22, Avenue Galilée**
**F-92350 Le Plessis Robinson(FR)**

(72) Inventeur: **Rossey, Guy**
**10, rue Paul Verlaine**
**F-78960 - Voisins le Bretonneux(FR)**
Inventeur: **Ugolini, Antonio**
**8, rue de Seine**
**F-78230 - Le Pecq(FR)**
Inventeur: **Chekroun, Isaas**
**25 bis, rue des Econdeaux**
**F-93800 - Epinay(FR)**
Inventeur: **Vartanian, Abkar**
**65, Boulevard Michelet**
**Hardicourt**
**F-78250 - Meulaan(FR)**
Inventeur: **Wick, Alexander**
**10, Boulevard des Plants**
**F-78860 - St Nom le Breteche(FR)**

(74) Mandataire: **Ludwig, Jacques et al**
**SYNTHELABO,**
**Service Brevets,**
**B.P. 72**
**F-92352 Le Plessis Robinson Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**JOURNAL OF CHEMICAL SOCIETY, PERKIN TRANSACTIONS I1985, pages 421-427; T. HASHIYAMA et al.: "Reaction of 3-phenylglycidic esters. Part 2. Stereo- and regio-selectivity in the oxirane ring opening of methyl trans-3-(4-methoxyphenyl)glycidate with various thiophenols and the effects of solvent and temperature"**

## Description

La présente demande de brevet a pour objet un procédé de préparation de (+)-(2S,3S) [amino-2 phényl)thio]-3 hydroxy-2 (méthoxy-4 phényl)-3 propionates de méthyle.

Ces composés sont des intermédiaires pour la synthèse stéréospécifique du Diltiazem (DCI) et de ses dérivés chlorés. Ils répondent à la formule générale (I), dans laquelle X représente un atome d'hydrogène ou de chlore.

Le procédé selon l'invention est illustré par le schéma ci-dessous.

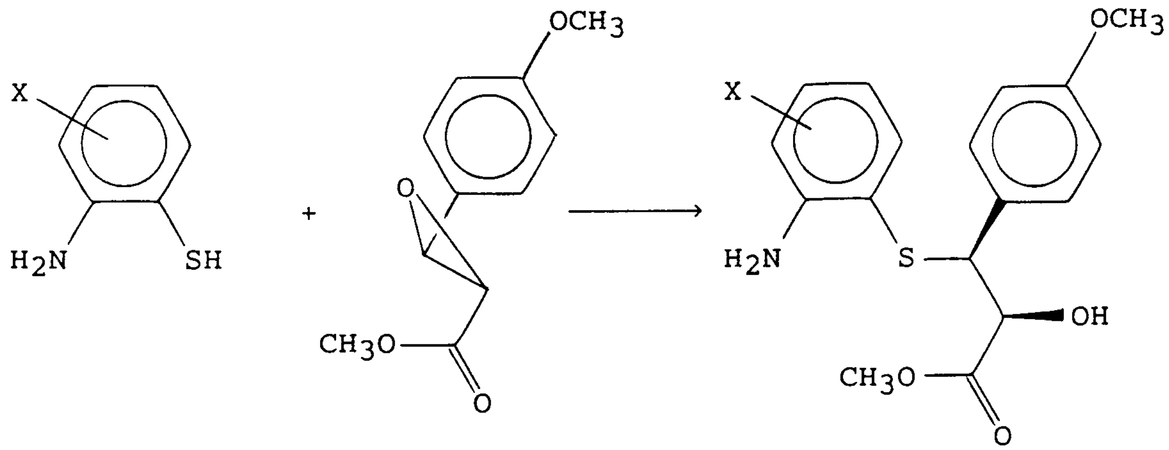

(II) (III) (I)

Il consiste à faire réagir un amino-2 thiophénol de formule générale (II) (dans laquelle X est tel que défini ci-dessus) avec le (-)-(2R,3S) (méthoxy-4 phényl)-3 glycidate de méthyle de formule (III), à chaud, sous atmosphère inerte, dans un solvant inerte.

Les composés de départ de la réaction sont décrits dans la littérature ; l'isomère (-)-(2R,3S) du glycidate, en particulier, est décrit dans les demandes de brevets japonais 145159/1986, 145160/1986 et 145174/1986.

Le principe de cette réaction a été maintes fois décrit dans la littérature, notamment à propos de procédés où intervient le glycidate racémique, et où le produit final est par conséquent un mélange d'isomères optiques.

Le problème était de pouvoir synthétiser un composé de formule (I), c'est-à-dire un composé de configuration (2S,3S) optiquement pur.

Or, il ne suffit pas, pour cela, d'utiliser l'énantiomère (2R,3S) du glycidate, car on obtient encore un mélange de diastéréoisomères, érythro et thréo, dont le rapport thréo/érythro est plus ou moins avantageux, et dont il faut isoler la forme thréo par une étape de procédé spécifique. Partant du plycidate optiquement pur, toute la difficulté consistait donc à obtenir, par action de l'amino-2 thiophénol, et avec un haut rendement, le diastéréoisomère thréo de l'aminoester possédant la stéréochimie relative désirée.

Les procédés connus mettent le plus souvent en oeuvre des solvants aromatiques (toluène, xylène, éthylbenzène), et ils conduisent à des rapport thréo/érythro peu intéressants.

C'est pourquoi un objet de la présente invention est d'offrir un procédé utilisable à l'échelle industrielle, et donnant un bon rapport thréo/érythro, sans que cet aspect avantageux soit atténué par des inconvénients tels que, par exemple, l'allongement de la durée de la réaction ou l'augmentation de la quantité d'énergie à fournir.

Cet objet a été atteint, conformément à l'invention, grâce à l'utilisation de dichloro-1,2 éthane comme solvant.

Un autre aspect avantageux du procédé de l'invention tient au fait qu'il est inutile d'ajouter un catalyseur à la réaction pour obtenir de hauts rendements, contrairement aux procédés connus. Cette caractéristique contribue à améliorer encore la pureté du produit final.

Enfin, il est à noter que l'[(amino-2 chloro-5 phényl)thio]-3 hydroxy-2 (méthoxy-4 phényl)-3 propionate de méthyle, de configuration (2S,3S), qui répond à la formule générale (I), est nouveau et fait partie de l'invention.

Les exemples qui vont suivre illustrent en détail le procédé selon l'invention.

Exemple 1

(+)-(2S,3S) [(Amino-2 phényl)thio]-3 hydroxy-2 (méthoxy-4 phényl)-3 propionate de méthyle.

Dans un ballon tricol de 2 l, maintenu sous azote, on introduit 200 g de (-)-(2R,3S) époxy-2,3 (méthoxy-4 phényl)-3 propionate de méthyle, 133 g d'amino-2 thiophénol et 900 ml de dichloro-1,2 éthane. On chauffe le mélange au reflux pendant 4 h 30, puis on élimine environ 400 ml de solvant par distillation. On ajoute 500 ml de cyclohexane et on laisse cristalliser le mélange à température ambiante pendant 2 h. Ensuite on le refroidit à 10 °C pendant 1 h, on le filtre, on lave le solide avec 200 ml d'un mélange 60/40 de cyclohexane/dichlorométhane et on le sèche à 60 °C sous vide pendant 12 h.
On recueille finalement 266,3 g de produit.
Point de fusion : 110 °C.
$[\alpha]_D^{20}$ = +294 ° (c = 0,5 ; MeOH).

Exemple 2.

(+)-(2S,3S) [(Amino-2 chloro-5 phényl)thio]-3 hydroxy-2 (méthoxy-4 phényl)-3 propionate de méthyle.

Dans un ballon de 50 ml, on place 1,5 g de (-)-(2R,3S) époxy-2,3 (méthoxy-4 phényl)-3 propionate de méthyle, 11,5 ml de dichloro-1,2 éthane et 1,18 g d'amino-2 chloro-5 thiophénol. On chauffe le mélange au reflux sous agitation pendant 4 h 30. On évapore le mélange réactionnel à sec sous vide, on chromatographie le résidu sur silice et on concentre. On obtient 1,4 g de produit que l'on dissout dans 5 ml d'acétate d'éthyle à chaud, puis on ajoute à la solution 10 ml de n-hexane. Le produit cristallise puis prend en masse. On laisse reposer au réfrigérateur pendant 3 h. On essore, lave avec de l'hexane, et sèche sous vide.
Point de fusion : 124-126 °C
$[\alpha]_D^{20}$ = + 244 ° (c = 0,5 ; MeOH).

## Revendications

1. Procédé de préparation de (+)-(2S,3S) [amino-2 phényl)thio]-3 hydroxy-2 (métoxy-4 phényl)-3 propionates de méthyle répondant à la formule générale (I)

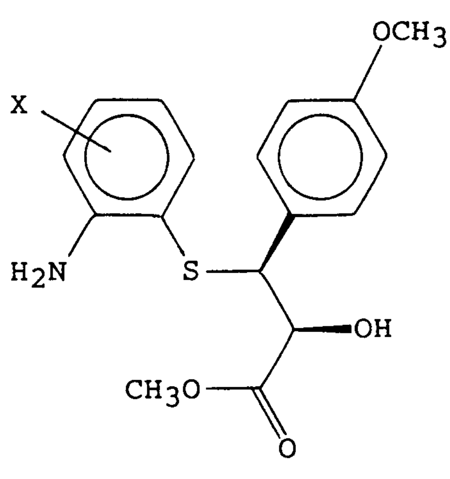

(I)

dans laquelle X représente un atome d'hydrogène ou de chlore, procédé caractérisé en ce qu'on fait réagir un amino-2 thiophénol de formule générale (II)

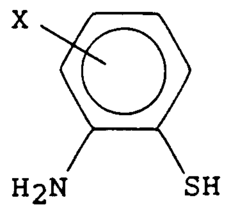

(II)

dans laquelle X est tel que défini ci-dessus, avec le (-)-(2R,3S) époxy-2,3 (méthoxy-4 phényl)-3 propionate de méthyle, en utilisant comme solvant le dichloro-1,2 éthane.

2. Procédé selon la revendication 1, caractérisé en ce qu'on opère en l'absence de catalyseur.

## Claims

1. Process for the preparation of methyl (+)-(2S,3S)-3-[(2-aminophenyl)thio]-2-hydroxy-3-(4-methoxyphenyl)propionates corresponding to the general formula (I)

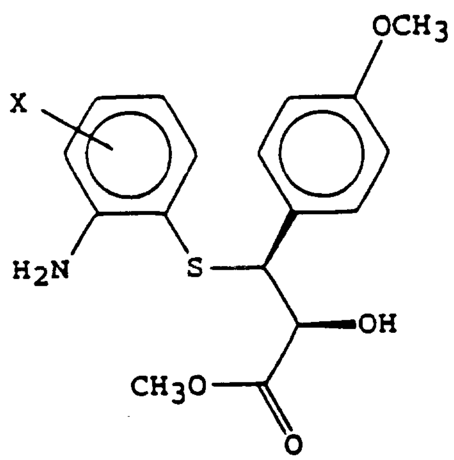

(I)

in which X denotes a hydrogen or chlorine atom, which process is characterized in that a 2-aminothiophenol of general formula (II)

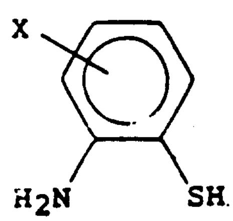

(II)

in which X is such as defined above, is reacted with methyl (-)-(2R,3S)-2,3-epoxy-3-(4-methoxyhenyl)-priopionate, using 1,2-dichloroethane as a solvent.

2. Process according to Claim 1, characterized in that the operation is carried out in the absence of a catalyst.

## Patentansprüche

1. Verfahren zur Herstellung von (+)-(2S,3S)-3-[(2-Amino-phenyl)-thio]-2-hydroxy-3-(4-methoxy-phenyl)-propionsäuremethylester der allgemeinen Formel (I)

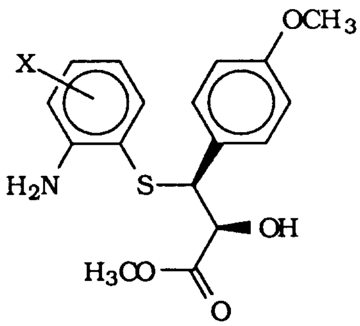

(I)

in der X ein Wasserstoffatom oder ein Chloratom bedeutet, **dadurch gekennzeichnet**, daß man ein 2-Amino-thiophenol der allgemeinen Formel (II)

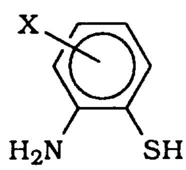

(II)

in der X die oben angegebenen Bedeutungen besitzt, mit (-)-(2R,3S)-2,3-Epoxy-3-(4-methoxy-phenyl)-propionsäuremethylester umsetzt, wobei man 1,2-Dichlorethan als Lösungsmittel verwendet.

2. Verfahren nach Anspruch 1, **dadurch gekennzeichnet**, daß man in Abwesenheit eines Katalysators arbeitet.